# EUROPEAN PATENT SPECIFICATION

(11) **EP 1 181 386 B1**
(45) Date of publication and mention of the grant of the patent: **11.02.2009**
(21) Application number: 00930502.0
(22) Date of filing: 10.05.2000
(51) Int. Cl.: C12Q 1/02, C12Q 1/70, G01N 33/573, A61K 49/00, A01N 63/00, C12Q 1/37

(54) **DETECTION OF VIRAL STABILITY**
NACHWEIS VON VIRUSSTABILITÄT
DETECTION DE STABILITE VIRALE

(30) Priority: 14.05.1999 US 134163 P
(43) Date of publication of application: 27.02.2002
(73) Proprietor: Merck & Co., Inc., Rahway, New Jersey 07065-0907 (US)
(72) Inventor: BLUE, Jeffrey, T., Rahway, NJ 07065-0907 (US)
(74) Representative: Horgan, James Michael Frederic
(86) International application number: PCT/US2000/012638
(87) International publication number: WO 2000/070081

(56) References cited:
- US-A- 5 972 899
- US-A- 5 976 822
- UMINO Y ET AL: "IMPROVEMENT IN POTENCY ASSAY OF MEASLES-MUMPS-RUBELLA TRIVALENT VACCINE INTERFERENCE BETWEEN COMPONENTS AND MEASURES FOR ITS ELIMINATION" JOURNAL OF VIROLOGICAL METHODS, vol. 27, no. 2, 1990, pages 159-168, XP009002598 ISSN: 0166-0934
- ESOLEN LISA M ET AL: "Apoptosis as a cause of death in measles virus-infected cells." JOURNAL OF VIROLOGY, vol. 69, no. 6, 1995, pages 3955-3958, XP002224405 ISSN: 0022-538X
- ITO MASAHIRO ET AL: "Detection of measles virus-induced apoptosis of human monocytic cell line (THP-1) by DNA fragmentation ELISA." FEMS IMMUNOLOGY AND MEDICAL MICROBIOLOGY, vol. 15, no. 2-3, 1996, pages 115-122, XP002224406 ISSN: 0928-8244
- DUNCAN ROBERT ET AL: "Rubella virus-induced apoptosis varies among cell lines and is modulated by Bcl-XL and caspase inhibitors." VIROLOGY, vol. 255, no. 1, 1 March 1999 (1999-03-01), pages 117-128, XP002224407 ISSN: 0042-6822
- SADZOT-DELVAUX CATHERINE ET AL: "Varicella-zoster virus induces apoptosis in cell culture." JOURNAL OF GENERAL VIROLOGY, vol. 76, no. 11, 1995, pages 2875-2879, XP009002370 ISSN: 0022-1317
- SHEN YUQIAO ET AL: "Viruses and apoptosis." CURRENT OPINION IN GENETICS & DEVELOPMENT, vol. 5, no. 1, 1995, pages 105-111, XP009002369 ISSN: 0959-437X
- MACEN J ET AL: "Activation of caspases in pig kidney cells infected with wild-type and CrmA/SPI-2 mutants of cowpox and rabbitpox viruses." JOURNAL OF VIROLOGY, vol. 72, no. 5, March 1998 (1998-03), pages 3524-3533, XP002224408 ISSN: 0022-538X
- "APO ALERT: CPP32 protease assay kits" CLONTECH ADVERTISSEMENT, [Online] XP002224410 Retrieved from the Internet: <URL:HTTP://WWW.CLONTECH.COM/ARCHIVE/JAN97 UPD/APOALERTC> [retrieved on 2003-01-21]
- CARTHY CHRISTOPHER M ET AL: "Caspase activation and specific cleavage of substrates after coxsackievirus B3-induced cytopathic effect in HeLa cells." JOURNAL OF VIROLOGY, vol. 72, no. 9, 1998, pages 7669-7675, XP002224409 ISSN: 0022-538X
- COHEN G: "Caspase: the executioners of apoptosis" BIOCHEMICAL JOURNAL, PORTLAND PRESS, LONDON, GB, vol. 326, 15 August 1997 (1997-08-15), pages 1-16, XP002091927 ISSN: 0264-6021

## Description

### FIELD OF THE INVENTION

The present invention pertains to assays for measuring viral potency and stability and for evaluating the stability of a virus in different formulations.

### BACKGROUND OF THE INVENTION

In developing live virus vaccines it is important to determine viral potency and stability to assure adequate immunization. The plaque assay is a common method for determining viral potency and stability. Other methods are described in Umino, et al., J. Virological Methods, 1990. The plaque assay is often used to examine the effect of different storage conditions on viral stability.

The plaque assay involves inoculating viruses onto a cell sheet at dilutions determined by the expected (estimated) potency. The plates containing the infected cells are incubated for a specified length of time and then stained. Viral infection results in plaque formation, or areas of dead or detached cells. Counting the number of plaques gives the corresponding plaque-forming units (PFU), a measure of potency. Viral stability is determined by examining the change in PFU over time.

### SUMMARY OF THE INVENTION

Viral induction of caspase 3 activity was found to provide a reliable measure of viral activity. Assaying viral induction of caspase 3 activity can be used, for example, in methods for measuring viral potency and stability, and for evaluating the stability of a virus in different formulations.

Thus, a first aspect of the present invention describes a method of assaying the potency and stability of a virus by measuring viral induction of caspase 3 activity. The method involves infecting a plurality of cells susceptible to caspase 3 induction with the virus and measuring caspase 3 activity as an indication of viral potency and stability. A plurality of cells is a population of the same strain of cells.

Another aspect of the present invention describes a method of identifying a stabilizing formulation for a virus. The method involves infecting a strain of cells susceptible to caspase 3 induction with a virus in different test formulations. The strain of cells is provided as different pluralities of cells where each plurality of cells is infected with the virus. Induction of caspase 3 activity produced from virus stored in the different formulations is measured to identify the formulations that stabilize the virus.

Other features and advantages of the present invention are apparent based on the descriptions provided herein. The examples provided herein illustrate different components and methodology useful in practicing the present invention. The examples do not limit the claimed invention. Based on the present disclosure the skilled artisan can identify and employ other components and methodology for practicing the present invention.

### BRIEF DESCRIPTION OF THE DRAWINGS

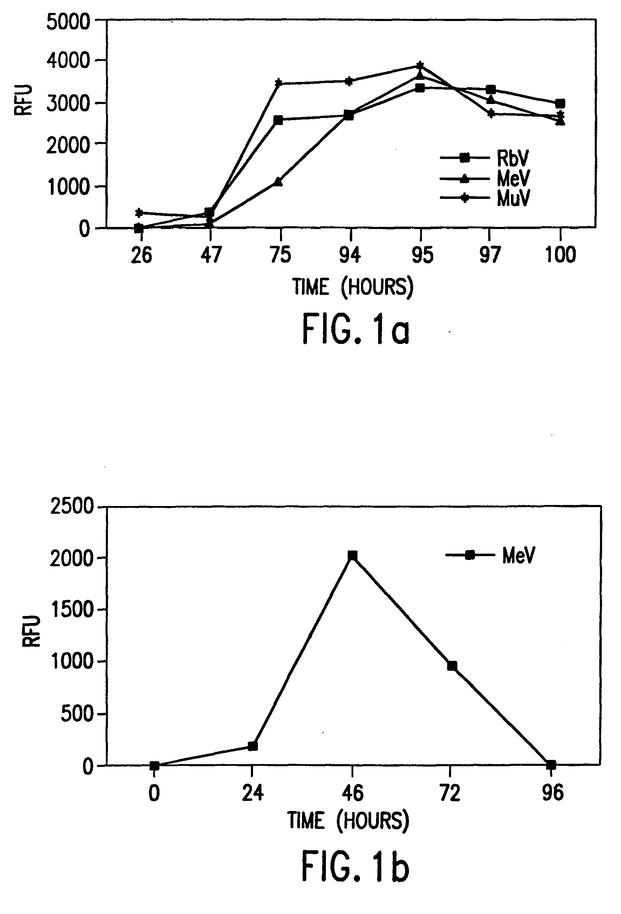

Figures 1a and 1b illustrate induction times for caspase 3 activity using lyophilized virus and liquid bulk virus (i.e., a concentrated lot of clarified virus). The RFU (relative fluorescent unit) value was adjusted by subtracting background signal (adjusted RFU). Figure 1a illustrates an induction time using lyophilized mumps virus ("MuV"), lyophilized rubella virus ("RbV"), and lyophilized measles virus ("MeV"). Figure 1b illustrates an induction time using bulk measles virus.

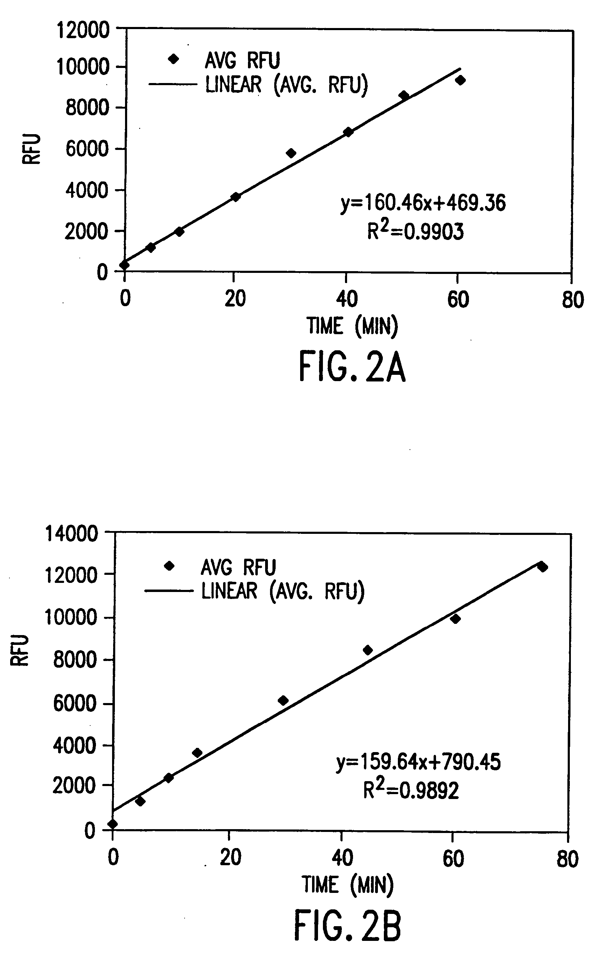

Figures 2a and 2b illustrate a linear range for induction of caspase 3 activity. Figure 2a provides results using measles virus. Figure 2b provides results using mumps virus.

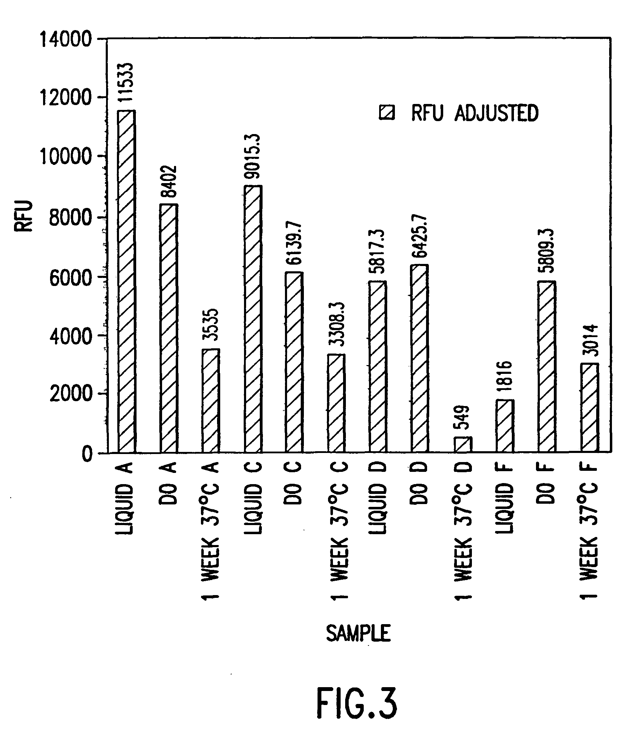

Figure 3 illustrates measles virus induction variation in various samples. "Liquid" indicates a non-lyophilized frozen aliquot. "D0" indicates a lyophilized non-incubated sample stored at -70°C. The one-week sample was initially lyophilized and stored at 37°C for one week. "A", "C", "D" and "F" refer to different formulations of the vaccine.

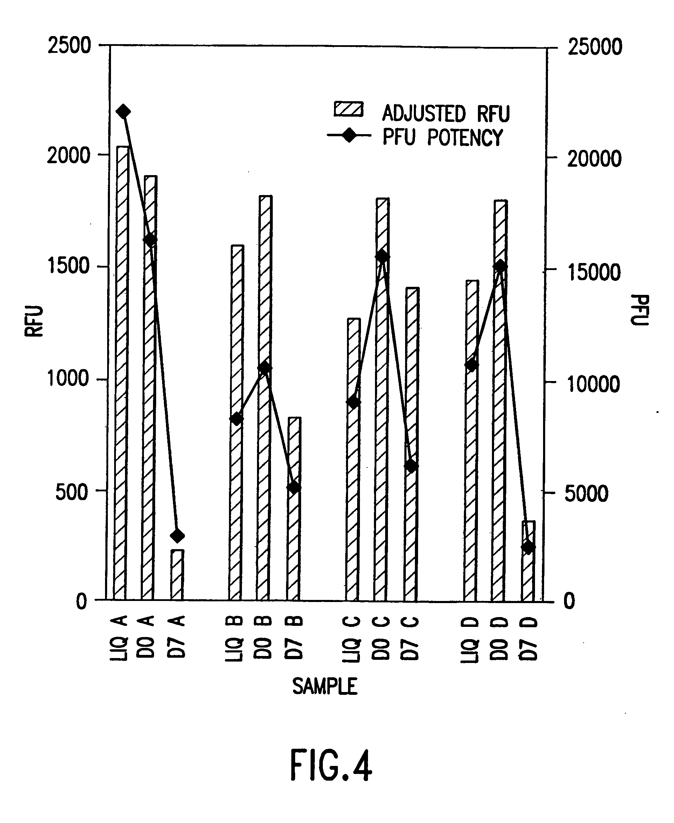

Figure 4 illustrates a comparison of the plaque assay and the caspase 3 assay. Activity was measured using measles virus stored in different environments. "Liq" indicates a non-lyophilized frozen aliquot. "D0" indicates a lyophilized, non-incubated sample stored at -70°C. "D7" indicates a lyophilized sample incubated at 37°C for one week. "A", "B", "C" and "D" refer to different formulations.

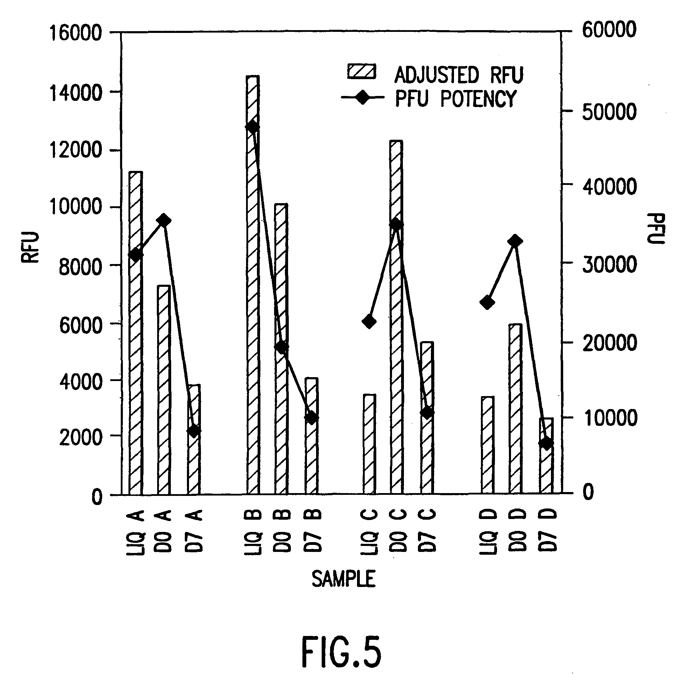

Figure 5 illustrates a comparison of the plaque assay and the caspase 3 assay. Viral activity was measured using mumps virus stored in different environments. "Liq" indicates a non-lyophilized frozen aliquot. "D0" indicates a lyophilized non-incubated sample stored at -70°C. "D7" indicates a lyophilized sample incubated at 37°C for one week. "A", "B", "C" and "D" refer to different vaccine formulations being tested.

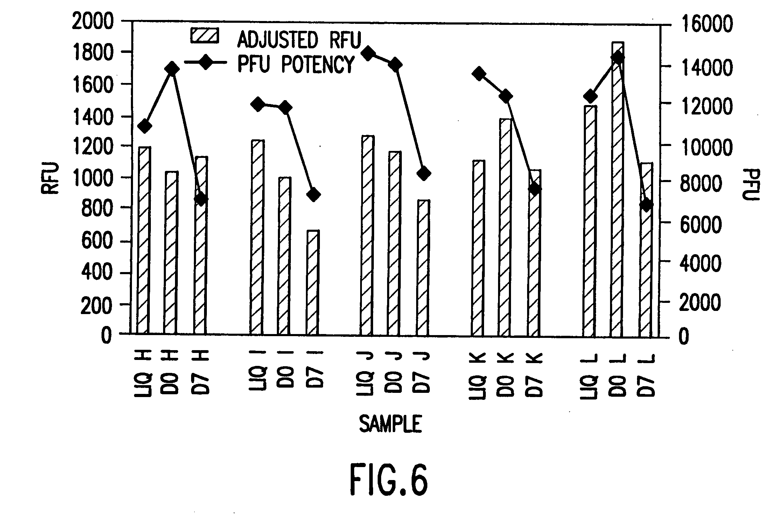

Figure 6 illustrates a comparison of the plaque assay and the caspase 3 assay. Viral activity was measured using rubella virus stored in different environments. "Liq" indicates non-lyophilized frozen aliquot. "D0" indicates a lyophilized, non-incubated sample stored at -70°C. "D7" indicates lyophilized sample incubated at 37°C for one week. "H", "I", "J", "K" and "L" refer to different formulations of the vaccine.

### DETAILED DESCRIPTION OF THE INVENTION

Measuring viral induction of caspase 3 activity was found to provide a reliable measure of viral potency and stability. Useful properties of measuring viral induction of caspase 3 activity include: induction of caspase 3 activity is achieved by different viruses including measles, mumps and rubella viruses; induction of caspase 3 activity is reproducible; induction of caspase 3 activity is sensitive to viral dilutions; induction of caspase 3 activity is sensitive to sample differences; and induction of caspase 3 activity correlates with the plaque assay. Thus, the caspase 3 assay can be applied to different formulations and a variety of viruses. Additionally, caspase 3 activity is not substantially effected by freezing and thawing, can be specifically detected, and is quenchable. These advantages allow the assay to be easily implemented for routine laboratory handling.

Another advantage of the caspase 3 assay in time. When measuring viral stability, the caspase 3 assay can be performed quicker than the plaque assay. The standard plaque assay takes approximately seven to ten days, but the caspase 3 assay can be performed in about half that time.

### Caspase 3

Caspase 3 (also known as CPP32 or apopain) is a member of the caspase family of proteases. Caspases are induced by apoptosis, which is an active process of cellular suicide. (Thompson, Science 267:1456-1462, 1995; Nicholson, Nature Biotechnology 14:297-301, 1996; Lincz, Immunology and Cell Biology 76:1-19, 1998.) Apoptosis has been indicated to be induced by different stimuli including infection by different viruses. (Shen, et al., Current Opinion in Genetic and Development 5:105-111, 1995; Sadzot-Delvaux, et al., Journal of General Virology 76:2875-2879, 1995; Esolen, et al., Journal of Virology 69:3955-3958, 1995; Ito, et al., FEMS Immunology and Medical Microbiology 15:115-122, 1996; and Duncan, et al., Virology 255:117-128, 1999.) However, certain viral proteins have been indicated to block apoptosis, such as Baculovirus protein p35 which inhibits caspase 3. (Lincz, Immunology and Cell Biology 76:1-19, 1998.)

Caspase 3 is a cysteine protease synthesized from a pro-enzyme by cleavage at an Asp residue to form an active protease. The active caspase 3 protease also cleaves at Asp residues. The specific recognition sequence for caspase 3 is the peptide Asp-Glu-Val-Asp. (Fernandes-Alnemri, et al., Journal of Biological Chemistry 269:30761-30764, 1994; Nicholson, et al., Nature 376:37-43, 1995; Srinivasula, et al, Journal of Biological Chemistry 271:27099-27106, 1996; Schlegel, et al., Journal of Biological Chemistry 271:1841-1844, 1996; and Casciola-Rosen, et al., J. Exp. Med. 183:1957-1964, 1996.

### Selection of Appropriate Viruses and Cells

Suitable virus and cells for use in the present invention can be selected based upon the susceptibility of a particular cell to caspase 3 induction when infected with a particular virus. The ability of a virus to infect a particular cell can readily be determined, as can the ability of the virus to induce caspase 3 activity. Examples of procedures that can be used to measure the suitability of a cell and a virus are described in the Example section provided below using measles, mumps and rubella virus. Other cells (e.g., MRC-5) and viruses (e.g., varicella-zoster) are expected to be useful in the present invention.

### Measuring Caspase 3 Activity

"Caspase 3 activity" refers to enzymatic activity able to cleave the caspase 3 substrate Asp-Glu-Val-Asp ("DEVD"). Such activity is known to be produced by caspase 3 and at least one related enzyme, Mch3α. (Fernandes-Alnemri, et al., Cancer Research 55:6045-6052, 1995.)

Based on the present disclosure, caspase 3 activity induced by viral infection can be measured using techniques well known in the art. (Macen, et al., J. Virology 72:3524-3533, 1998.) Preferably, caspase 3 activity is measured using colorimetric or fluorimetric labeled substrates. (ApoAlert™ CPP32 Protease Assay Kits, CLONTECHnique, 1997.) More preferably, the employed substrate is DEVD linked to a colorimetric or fluorimetric moiety. Examples of such a moiety include the colorimetric moiety p-nitroanilide (λₘₐₓ = 505 nm) and the fluorimetric moiety 7-amino-4-trifluoromethyl coumarin ("AFC", λₘₐₓ = 400 nm). (Zhang, et al., in Apoptosis Detection and Assay Methods, pages 7-14, Eds. Zhu and Chun, BioTechniques Books, 1998.) Colorimetric and fluorimetric labeled substrates can be employed using procedures such as those described by Zhang, et al.; in Apoptosis Detection and Assay Methods, pages 7-14, Eds. Zhu and Chun, BioTechniques Books, 1998; and ApoAlert^{™} CPP32/Caspase-3 Assay Kits User Manual (PT3083-1), CLONTECH Laboratories, Inc. 1998.

The caspase 3 assay is preferably employed on viral vaccine samples either in liquid or lyophilized form. For example, lyophilized viral samples can be reconstituted, diluted, and plated onto a cell sheet; the samples are incubated, the cells are lysed, and the cellular lysate collected and frozen at -70°C; after thawing on wet ice (about 5°C) removal of cellular debris by centrifugation occurs, the supernatant is removed to a new tube containing reaction buffer, and the DEVD-AFC substrate is incubated with the cellular supernatant.

### EXAMPLES

Examples are provided below to further illustrate different features and advantages of the present invention. The examples also illustrate useful methodology for practicing the invention. These examples do not limit the claimed invention.

### Methods and Materials

Methods and materials generally used in growing cells, inducing caspase 3 activity, and measuring caspase 3 activity to obtain the results provided below were as follows:

### Growth of Vero Cells and RK-13 Cells

Vero cells were grown to confluency in 162 cm² flasks in M199 media (Gibco/BRL cat# 11150-059) containing 10% fetal bovine serum (FBS) and 0.5% neomycin at 35°C with 5% CO₂. The flasks were then decanted and 3 ml of 1x trypsin-ethylene diamine tetra acetic acid (EDTA) (Gibco/BRL cat# 25200-056) was added to the flasks to neutralize the media. The trypsin was decanted and another 2 mls of trypsin was added and incubated for 10 to 15 minutes at 35°C with 5% CO₂. Cells were then washed to the bottom of the flask with 20 ml of M199 containing 10% FBS and 0.5% neomycin.

Cells were plated into 24 well plates at 5.4 X 10⁴ cells/ml. Each well received 2 ml of the diluted cells (diluted into M199 containing 10% FBS and 0.5% neomycin). The plates were incubated at 35°C for 2 days and then used for caspase 3 induction. The growth of RK-13 cells is the same as Vero cells with the exception of the media. RK-13 cells were grown in MEM media (Gibco/BRL) containing 10% FBS and 0.5% neomycin. The cells were plated at a density of 6.0 x 10⁴ cells /ml, 2 ml per well.

### Induction of Caspase 3 Activity

Generally, Vero plates were used for measles virus and mumps virus infections, while RK-13 cells were used for the rubella virus infections. Lyophilized formulations of a combination of measles, mumps, and rubella vaccine were used. Lyophilized formulations were reconstituted with 0.7 ml dH₂O. Frozen liquid samples were thawed at room temperature prior to use. Following reconstitution or liquid thawing, the samples were diluted 1:2, 1:5, 1:10 or 1:20 in their respective diluents. The diluents contained antibodies against the viruses not being assayed for among mumps, measles, and rubella viruses (e.g., when assaying for measles virus antibodies for mumps and measles virus were used).

Cells (2.0x10⁵) were infected with 50 µl of the diluted samples. Each sample was used to infect 6 wells on a 24 well plate. Plates were then incubated at 35°C with 5% CO₂ for 1 hr for plates to be assayed for mumps and rubella viruses and for 2 hrs for plates to be assayed for measles virus. During this attachment phase, the plates were rocked every 15 minutes to ensure proper coverage of the cell sheet with media. Following the attachment phase, the cells were overlayed with 1 ml of M199 media containing 10% FBS and 0.5% neomycin for the Vero cells and 1 ml of MEM containing 10% FBS and 0.5% neomycin for the RK-13 cells. The plates were then incubated at 35°C with 5% CO₂ for 93 to 96 hrs.

### Caspase 3 Assay

Following the induction period, media was aspirated from the wells. Thirty microliters of chilled cell lysis buffer (Clontech: 20 mM Tris-HCl, pH 7.5, 150 mM NaCl, 1% Triton X -100) was added per well. The samples were incubated on ice for 10 minutes and then two wells were pooled together into one microfuge tube. Samples were either frozen on dry ice and stored at -70°C until time of analysis or were assayed immediately.

The samples were spun in at 12,000 rpm for 5 minutes at 4°C. Supernatant (50 µl) was removed to a new microfuge tube and 50 µl of 2x Reaction Buffer (Clontech: 100 mM HEPES, pH 7.4, 150 mM NaCl, 0.2% CHAPS) containing 7 mM dithiothreitol (DTT) was added. Five microliters of the 1 mM DEVD-AFC substrate was added to the reaction and vortexed briefly. The samples were then incubated for 1 hr at 37°C in a circulating water bath. Following the incubation, 20 µl of 10% H₃PO₄ was used to quench the reaction. Quenched reactions (100 µl) were added to a 96 well plate and read in a TECAN fluorescent plate reader (excitation: 390 nm, emission: 480 nm, gain: 60).

### Results

### Detection of Caspase 3 Activity Induced bv Measles, Mumps and Rubella Viruses

The ability of measles, mumps, and rubella viruses to induce caspase 3 activity in cells was confirmed using Vero and RK-13 cells. Caspase 3 activity was measured as described above in Methods and Materials. Vero cells were induced with measles virus, mumps virus, and rubella virus, while RK-13 cells were induced with rubella virus. The results are shown in Table 1

**Table 1**

| | Induced | Non-Induced |
|---|---|---|
| Measles Virus | 12861 | 1210 |
| Mumps Virus | 20085 | 1286 |
| Rubella Virus | 4060 | 554 |

Caspase 3 activity expressed as RFU in induced (*i.e.* infected) cells and non-induced controls.

To ensure that the observed signal was due to the virus, one set of Vero cells were infected with measles virus and another set of Vero cells were infected with a placebo. The placebo produced an adjusted RFU of 50-500, while measles virus produced an adjusted RFU of about 7,750. These results demonstrate that significant changes in caspase 3 activity are due to viral infection rather than a formulation component.

### Effects of Freeze-Thawing on Caspase 3 Activity

To allow an assay to be run at various times after infected, samples may be frozen and later thawed at a convenient time. To determine if freezing and thawing affects caspase 3 activity, samples were frozen on dry ice and stored at -70°C following cell lysis. The samples were then thawed and assayed with a set of samples that were not frozen. Non-frozen cells provided an adjusted RFU of 2,167 while frozen cells produced an adjusted RFU of 2,175.5. Thus, freeze-thawing does not appear to effect caspase 3 activity. Accordingly, many samples can be run and then stored for various lengths of time allowing the actual assay to be run at a later time.

### Determination of the Optimal Time Required for Viral Bulk Incubation

The time required to induce caspase 3 activity with various samples can differ depending on the virus and multiplicity of infection (MOI). To determine preferred times for measles, mumps and rubella virus infection, Vero cells or RK-13 cells were infected with (1) measles virus bulk and (2) different lyophilized measles virus, mumps virus, and rubella virus. Vero cells were infected with mumps and measles, while RK-13 cells were infected with rubella virus. A long-term study was initiated with time points collected at 24, 46, 72, and 96 hours for bulk samples and 26, 47, 75, 94, 95, 97 and 100 hours for lyophilized samples.

The results of different points are presented in Figures 1a and 1b. All three viruses in lyophilized formulations show a peak signal around 95 hours (Figure 1a). The optimal signal observed for the infection with measles virus bulk was around 46 hours (Figure 1b). This difference in overall time is expected since a bulk sample contains a much higher titer and infects more cells upon inoculation, thereby decreasing the length of time necessary to ensure an optimal signal.

### Linearity of the Caspase 3 Enzyme Response from Cells induced with Measles Virus and Mumps Virus

When investigating and comparing enzyme activity, it is important to collect data found within the linear range of the assay. Following induction with measles and mumps viruses, the samples were assayed and data collected over a one-hour period for the measles virus and an hour and a half for mumps virus. The data presented in Figure 2a (measles virus) shows the assay is linear for at least one hour, and the data presented in Figure 2b (mumps virus) show the assay is linear for at least 75 minutes. Thus, using a one-hour incubation period for caspase 3 enzyme activity will ensure the data collected will be meaningful.

### Determination of Non-Specific Substrate Cleavage

A concern with using a non-purified cell lysate is the effect of non-specific substrate cleavage derived from other proteases found within the lysate. Using a specific inhibitor for caspase 3, DEVD-CHO, the caspase 3 assay was run and analyzed. If non-specific cleavage were occurring in the assay, the addition of the inhibitor would not completely stop the substrate from being cleaved. The results shown in Table 2 indicate that adding 1 µM DEVD-CHO inhibitor eliminates the RFU signal.

**TABLE 2**

| Virus | - Inhibitor (RFU) | + Inhibitor (RFU) |
|---|---|---|
| Measles Virus | 2538 | 6 |
| Rubella Virus | 3205 | 10 |
| Mumps Virus | 3074 | 0 |

| | | |
|---|---|---|
| RFU was adjusted to take background into account. | | |

Samples without the caspase 3 inhibitor show a -3,000 fold increase over the samples containing the inhibitor. Thus, non-specific cleavage is not occurring in these cellular lysates and the signal is derived from caspase 3 activity.

### The Effects of Quenching Caspase 3 Assay using 10% H₃PO₄

To ensure consistent data when using enzymes, it is useful to be able to quench the reaction. Quenching can be used to ensure that each sample is incubated for the same amount of time. It is useful to examine the quenching conditions to ensure that the quench is not having an effect on the RFU signal that is being generated. In some instances, quenching a fluorescent peptide will provide interference on the overall signal making data interpretation difficult.

Following a one hour incubation period, samples were quenched with 20 µl of 10% H₃PO₄ and analyzed on the TECAN Plate reader. Data were compared with that obtained from samples that did not undergo quenching. The results are shown in Table 3.

**TABLE 3**

| | Not Quenched (RFU) | Quenched (RFU) | Non-Induced Not Quenched (RFU) | Non-Induced Quenched |
|---|---|---|---|---|
| Measles Virus | 9331 | 5069 | 1046 | 508 |

The results indicate the ratio of the non-induced samples to the induced samples is the same. Thus, quenching the reactions does not effect the relative signal intensity.

### Reproducibility of the Caspase 3 Assay

Using samples induced by measles virus, mumps virus, or rubella virus, the reproducibility of measuring caspase 3 activity was examined by using three vials of the same sample. The data presented in Table 4 show that the assay is reproducible and very consistent.

**TABLE4**

| | Sample 1 (RFU) | Sample 2 (RFU) | Sample 3 (RFU) |
|---|---|---|---|
| Measles Virus | 4332 | 4372 | 3694 |
| Mumps Virus | 11377 | 12621 | 11454 |
| Rubella Virus | 3248 | 3166 | 3199 |

| | | | |
|---|---|---|---|
| RFU was adjusted to take into account the background. | | | |

### Effects of Dilutions on Caspase 3 Signal

To produce a quantitative assay it is important to detect differences in signal when the sample is diluted to different multiplicity of infections. To address this, measles virus and rubella virus lyophilized samples were diluted 1:2, 1:5 and 1:10 in their respective diluents, and mumps virus was diluted 1:20, 1:40, and 1:80, and the effect on caspase 3 signal was measured. The results are shown in Tables 5 and 6.

**TABLE 5**

| | 1:2 Dilution (RFU) | 1:5 Dilution (RFU) | 1:10 Dilution (RFU) |
|---|---|---|---|
| Measles Virus | 8221 | 3112 | 1441 |
| Rubella Virus | 3184 | 1721 | 799 |

| | | | |
|---|---|---|---|
| RFU was adjusted to take into account the background. | | | |

**TABLE 6**

| | 1:20 Dilution (RFU) | 1:40 Dilution (RFU) | 1:80 Dilution (RFU) |
|---|---|---|---|
| Mumps Virus | 13681 | 9524 | 5061 |

| | | | |
|---|---|---|---|
| RFU was adjusted to take into account the background. | | | |

Dilution effects were observed for all three viruses. This is observed by the overall decrease in signal as the dilution is increased. Thus, the induction of caspase 3 activity is affected by the multiplicity of infection used to infect the cells. Because signal output is affected through dilutions, it is important to have the same multiplicity of infection for direct comparisons between vaccine formulations.

### Detection of Differences in Signal Derived from Various Lyophilized Samples

The ability of the caspase 3 assay to detect the effect of different sample and storage conditions was measured to determine the suitability of using the caspase 3 assay to measure the stability of viral preparations stored under different conditions. Measles virus was used to infect cells.

The data shown in Figure 3 illustrates that the effect of different conditions on viral stability can be measured by measuring caspase 3 activity. Thus, the caspase 3 assay can detect differences in viral potency and stability due to various formulation composition and storage conditions.

### Correlation Between the Plaque and Caspase 3 Assays

A stability study was performed to examine the correlation between the plaque and caspase 3 assays using measles virus, mumps virus, and rubella virus. The data shown in Figures 4-6 illustrate that as the PFU potency decreased, the corresponding caspase 3 signal also decreased.

Other embodiments are within the following claims.

## Claims

1. A method for assaying potency and stability of a virus comprising the steps of:
a) contacting a plurality of cells susceptible to caspase 3 induction with said virus; and
b) measuring caspase 3 activity as an indication of viral stability.

2. The method of claim 1, wherein said caspase 3 activity is measured using a caspase 3 substrate linked to a fluorimetric or a colorimetric moiety.

3. The method of claim 2, wherein said substrate is the peptide Asp-Glu-Val-Asp.

4. The method of claim 3, wherein said virus is either measles virus, mumps virus, or rubella virus.

5. The method of claim 4, wherein said plurality of cells is either Vero cells or RK-13 cells.

6. The method of claim 3, wherein prior to said step (a) said virus was lyophilized.

7. The method of claim 3, wherein said step (a) and said step (b) are performed at two or more time intervals.

8. The method of claim 3, wherein after said step (a) and prior to said step (b) said cells were frozen and then thawed.

9. A method of identifying a stabilizing formulation comprising the steps of:
(a) assaying a virus stored in a first test solution with a first plurality of cells according to the method of any one of claims 1 to 8, and assaying the virus stored in a second test formulation with a second plurality of cells according to the method of any one of claims 1 to 8 wherein the first and second plurality of cells are the same strain and are susceptible to caspase 3 inductions; and
(b) measuring caspase 3 activity produced from said virus stored in said first and said second test formulation to identify said stabilizing formulation.

10. The method of claim 9 wherein step (a) further comprises assaying the virus stored in a third test formulation with a third plurality of cells according to the method of any one of claims 1 to 8, and assaying the virus stored in a fourth test formulation with a fourth plurality of cells according to any one of claims 1 to 8, wherein said third and fourth plurality of cells are the same strain as said first and second plurality of cells.

## Patentansprüche

1. Ein Verfahren zur Bestimmung der Wirksamkeit und Stabilität eines Virus, umfassend die Schritte:
a) Inkontaktbringen einer Vielzahl von gegen Caspase-3-Induktion empfindlichen Zellen mit dem Virus und
b) Messen der Caspase-3-Aktivität als ein Anzeichen für die Virusstabilität.

2. Das Verfahren nach Anspruch 1, bei dem die Caspase-3-Aktivität unter Verwendung eines Caspase-3-Substrats gemessen wird, das an einen fluorimetrischen oder kolorimetrischen Rest gebunden ist.

3. Das Verfahren nach Anspruch 2, bei dem das Substrat das Peptid Asp-Glu-Val-Asp ist.

4. Das Verfahren nach Anspruch 3, bei dem das Virus entweder Masernvirus, Mumpsvirus oder Rötelvirus ist.

5. Das Verfahren nach Anspruch 4, bei dem die Vielzahl von Zellen entweder Vero-Zellen oder RK-13-Zellen ist.

6. Das Verfahren nach Anspruch 3, bei dem vor dem Schritt (a) das Virus gefriergetrocknet wurde.

7. Das Verfahren nach Anspruch 3, bei dem der Schritt (a) und der Schritt (b) in zwei oder mehreren Zeitintervallen durchgeführt werden.

8. Das Verfahren nach Anspruch 3, bei dem nach dem Schritt (a) und vor dem Schritt (b) die Zellen eingefroren und dann aufgetaut wurden.

9. Ein Verfahren zur Identifizierung einer stabilisierenden Formulierung, umfassend die Schritte:
(a) Bestimmung eines in einer ersten Testlösung aufbewahrten Virus mit einer ersten Vielzahl von Zellen gemäß dem Verfahren nach irgendeinem der Ansprüche 1 bis 8 und Bestimmung des in einer zweiten Testformulierung aufbewahrten Virus mit einer zweiten Vielzahl von Zellen gemäß dem Verfahren nach irgendeinem der Ansprüche 1 bis 8, wobei die erste und zweite Vielzahl von Zellen vom selben Stamm sind und gegen Caspase-3-Induktionen empfindlich sind, und
(b) Messung der Caspase-3-Aktivität, die von dem in der ersten und in der zweiten Testformulierung aufbewahrten Virus erzeugt wird, um die stabilisierende Formulierung zu identifizieren.

10. Das Verfahren nach Anspruch 9, wobei Schritt (a) ferner die Bestimmung des in einer dritten Testformulierung aufbewahrten Virus mit einer dritten Vielzahl von Zellen gemäß dem Verfahren nach irgendeinem der Ansprüche 1 bis 8 und die Bestimmung des in einer vierten Testformulierung aufbewahrten Virus mit einer vierten Vielzahl von Zellen gemäß irgendeinem der Ansprüche 1 bis 8 umfasst, wobei die dritte und vierte Vielzahl von Zellen vom selben Stamm wie die erste und zweite Vielzahl von Zellen sind.

## Revendications

1. Procédé de dosage de la puissance et de la stabilité d'un virus, comprenant les étapes consistant:
a) à mettre en contact une pluralité de cellules sensibles à une induction par caspase 3 avec ledit virus; et
b) à mesurer l'activité de caspase 3 en tant qu'indication de la stabilité virale.

2. Procédé selon la revendication 1, dans lequel ladite activité de caspase 3 est mesurée en utilisant un substrat pour caspase 3 lié à un groupe fluorimétrique ou colorimétrique.

3. Procédé selon la revendication 2, dans lequel ledit substrat est le peptide Asp-Glu-Val-Asp.

4. Procédé selon la revendication 3, dans lequel ledit virus est le virus de la rougeole, le virus des oreillons ou bien le virus de la rubéole.

5. Procédé selon la revendication 4, dans lequel ladite pluralité de cellules sont soit des cellules Vero soit des cellules RK-13.

6. Procédé selon la revendication 3, dans lequel, avant ladite étape (a), ledit virus est lyophilisé.

7. Procédé selon la revendication 3, dans lequel ladite étape (a) et ladite étape (b) sont effectuées à deux intervalles de temps ou plus.

8. Procédé selon la revendication 3, dans lequel, après ladite étape (a) et avant ladite étape (b), lesdites cellules sont congelées puis décongelées.

9. Procédé d'identification d'une formulation stabilisante, comprenant les étapes consistant à:
(a) à doser un virus stocké dans une première solution d'essai avec une première pluralité de cellules d'après le procédé selon l'une quelconque des revendications 1 à 8, et à doser le virus stocké dans une seconde formulation d'essai avec une seconde pluralité de cellules d'après le procédé selon l'une quelconque des revendications 1 à 8, dans lequel les première et seconde pluralités de cellules sont la même souche et sont sensibles à une induction par caspase 3; et
(b) à mesurer l'activité de caspase 3 produite par ledit virus stocké dans ladite première et ladite seconde formulation d'essai pour identifier ladite formulation stabilisante.

10. Procédé selon la revendication 9, dans lequel l'étape (a) comprend en outre le dosage du virus stocké dans une troisième formulation d'essai avec une troisième pluralité de cellules d'après le procédé selon l'une quelconque des revendications 1 à 8, et le dosage du virus stocké dans une quatrième formulation d'essai avec une quatrième pluralité de cellules selon l'une quelconque des revendications 1 à 8, dans lequel lesdites troisième et quatrième pluralités de cellules sont la même souche que lesdites première et seconde pluralités de cellules.
